# EUROPÄISCHE PATENTSCHRIFT

(11) **EP 1 749 009 B1**
(45) Veröffentlichungstag und Bekanntmachung des Hinweises auf die Patenterteilung: **06.08.2008**
(21) Anmeldenummer: 05740694.4
(22) Anmeldetag: 13.05.2005
(51) Int. Cl.: C07D 487/08

(54) **VERFAHREN ZUR HERSTELLUNG EINER LÖSUNG VON REINEM TRIETHYLENDIAMIN (TEDA)**
METHOD FOR PRODUCING A SOLUTION OF PURE TRIETHYLENEDIAMINE (TEDA)
PROCEDE DE PRODUCTION D'UNE SOLUTION DE TRIETHYLENEDIAMINE (TEDA) PURE

(30) Priorität: 15.05.2004 DE 102004024274
(43) Veröffentlichungstag der Anmeldung: 07.02.2007
(73) Patentinhaber: BASF SE, 67056 Ludwigshafen (DE)
(72) Erfinder: LANG, Ortmund, 66909 Quirnbach (DE); FRAUENKRON, Matthias, 67251 Freinsheim (DE)
(86) Internationale Anmeldenummer: PCT/EP2005/005226
(87) Internationale Veröffentlichungsnummer: WO 2005/111043

(56) Entgegenhaltungen:
- EP-A- 1 070 717
- CH-A5- 659 778

## Beschreibung

Die vorliegende Erfindung betrifft ein Verfahren zur Herstellung einer Lösung von reinem Triethylendiamin (= TEDA = DABCO® = 1,4-Diazabicyclo-[2,2,2]-octan), in dem man TEDA verdampft, das dampfförmige TEDA in ein flüssiges Lösungsmittel 1 einleitet (Quench) und das TEDA aus der erhaltenen Lösung auskristallisiert und abtrennt (Fest-Flüssig-Trennung).

TEDA ist ein wichtiges Zwischen- und Endprodukt in der chemischen Industrie, das hauptsächlich als solches bei der Polyurethanherstellung als Katalysator eingesetzt wird.

Für diese und andere Einsatzgebiete ist eine reine, möglichst geruchlose TEDA-Lösung mit einer möglichst geringen Verfärbung, z.B. mit einer möglichst kleinen APHA-Farbzahl (DIN-ISO 6271), die diese Eigenschaften auch über längere Lagerzeiten (von z.B. 6, 12 oder mehr Monaten) beibehält, erwünscht.

Zur Herstellung von Triethylendiamin (TEDA) existiert eine große Anzahl verschiedener Synthesen, die sich hauptsächlich in der Wahl der Edukte und der benutzten Katalysatoren unterscheiden.

Bei der Zeolith-katalysierten TEDA-Synthese werden hauptsächlich N-(2-aminoethyl)-piperazin (Tosoh Corp., JP-B-3 132 061, JP-B-3 132 062, JP-B-3 132 063 und EP-A1-1 192 993), Ethylendiamin und/oder Piperazin (Air Products, EP-A1-842 936; BASF AG, EP-A1-382 055, WO 01/02404, EP-A1-1 215 211 und WO 03/004499) als Einsatzstoffe verwendet. Mit dieser Fahrweise können Selektivitäten an TEDA von bis zu 90 % erreicht werden.

Die bekannten Verfahren zur TEDA-Herstellung führen zur Bildung roher Umsetzungsprodukte, die neben TEDA noch Wasser, Nebenprodukte, wie z.B. Piperazin und hochmolekulare Polymerisate, sowie ein gegebenenfalls bei der Umsetzung eingesetztes Lösungsmittel enthalten können. TEDA wird aus diesen Gemischen gewöhnlich durch diskontinuierliche oder kontinuierliche Destillation oder Rektifikation abgetrennt und meist in einem anschließenden Schritt durch Kristallisation oder Umkristallisation gereinigt.

TEDA ist aufgrund seiner Eigenschaften [hygroskopisch, temperaturempfindlich, Siedepunkt (174°C bei Normaldruck) und Schmelzpunkt (158-160°C) liegen dicht beieinander] schwierig und nur unter entsprechendem technischem Aufwand zu handhaben, ohne dass eine Verschlechterung der Qualität des TEDAs bezüglich Farbe, Farbstabilität (unerwünschte Zunahme der Farbzahl, z.B. gemessen als APHA-Farbzahl, über die Lagerzeit), Geruch (z.B. unerwünschter Geruch nach cyclischen gesättigten 5-Ring-N-Heterocyclen oder anderen cyclischen gesättigten 6-Ring-N-Heterocyclen und/oder aromatischen 5- oder 6-Ring-N-Heterocyclen) und Reinheit auftritt. Entsprechendes gilt für TEDA-Lösungen.

Die ältere deutsche Patentanmeldung Nr. 10303696.2 vom 30.01.03 (BASF AG) betrifft ein Verfahren zur Herstellung von TEDA-Lösungen enthaltend ein Lösungsmittel aus der Gruppe der mehrwertigen Alkohole und Ether von mehrwertigen Alkoholen, umfassend a) Einleiten von gasförmigem TEDA in das Lösungsmittel und b) Behandeln der Lösung mit einem oder mehreren geeigneten Adsorbentien.

EP-A1-1 070 717 (BASF AG) betrifft ein Verfahren zur Herstellung einer Lösung von reinem TEDA, wobei man TEDA verdampft und das dampfförmige TEDA in ein flüssiges Lösungsmittel 1 einleitet (Quench), sowie ein Verfahren zur Herstellung von reinem TEDA, indem man das TEDA aus dieser Lösung auskristallisiert.
Das kristalline TEDA wird in einer Fest-Flüssig-Trennung von dem beladenen Lösungsmittel 1 (Mutterlauge) abgetrennt und z.B. anschließend in einem zur weiteren Verarbeitung des TEDAs geeigneten zweiten Lösungsmittel (Lösungsmittel 2) aufgelöst.

EP-A-1 223 172, EP-A-1 258 485 und WO-A-03/022851 (alle BASF AG) betreffen weitere Ausgestaltungen des o.g. Quenchverfahrens zur Herstellung von TEDA-Lösungen bzw. reinem TEDA.

Nachteilig an diesen Verfahren ist, dass das gemäß den obigen Verfahren erhaltene kristalline TEDA noch mit Lösungsmittel 1 und darin gelösten Nebenkomponenten verunreinigt sein kann. Die möglichst vollständige Abtrennung des Lösungsmittels 1 vom kristallinen TEDA ist für die Einhaltung der geforderten Spezifikation unbedingt erforderlich.

Der vorliegenden Erfindung lag die Aufgabe zugrunde, ein verbessertes, effizientes und wirtschaftliches Verfahren zur Herstellung von reinen TEDA-Lösungen aufzufinden, die eine verbesserte Qualität bezüglich Farbe, Farbstabilität, Geruch und Reinheit aufweisen. Auch die Ausbeute bezüglich TEDA sollte dabei erhöht werden.

Erfindungsgemäß wurde erkannt, dass das Lösungsmittel 1 aus der Kristallisation und Neben- sowie Zersetzungsprodukte, die im Lösungsmittel 1 gelöst sind, zur Qualitätsminderung des TEDAs führen. Eine Qualitätsminderung des TEDAs kann dadurch verhindert werden, dass der größte Teil des Lösungsmittels 1 und damit auch die unerwünschten Nebenkomponenten abgetrennt werden.

Üblicherweise wird das noch enthaltene Lösungsmittel 1 in einem Trocknungsschritt (z.B. in einem Trockner) von TEDA abgetrennt. Dabei kommt es aufgrund der starken Sublimationsneigung zu einem erheblichen Produktverlust. In Abhängigkeit des verwendeten Lösungsmittels 1 nimmt mit zunehmender Temperatur und steigender Trockengasmenge der TEDA-Verlust zu. Um das Lösungsmittel 1 nahezu vollständig zu entfernen, müssen abhängig vom Lösungsmittel 1 hohe Temperaturen in der Trockenstufe eingestellt werden. Bei ca. 80°C kommt es zu einem aus der Literatur bekannten Übergang von LT (low-temperature) zu HT- (high-temperature) TEDA, welches zu sehr starker Verbackung neigt, so dass eine weitere Verarbeitung des TEDAs sehr schwierig wird.
Außerdem kann das bei der Kristallisation in den TEDA-Kristallen eingeschlossene Lösungsmittel 1 und damit auch die eingeschlossenen unerwünschten Nebenkomponenten nicht durch übliche Trocknungsverfahren entfernt werden.

Demgemäss wurde ein Verfahren zur Herstellung einer Lösung von reinem TEDA, in dem man TEDA verdampft, das dampfförmige TEDA in ein flüssiges Lösungsmittel 1 einleitet (Quench) und das TEDA aus der erhaltenen Lösung auskristallisiert und abtrennt (Fest-Flüssig-Trennung), gefunden, welches dadurch gekennzeichnet ist, dass man das erhaltene kristalline TEDA.in einem Lösungsmittel 2 löst und durch die resultierende Lösung ein Strippgas leitet (Strippung).

Gemäß dem erfindungsgemäßen Verfahren wird durch das Lösen der TEDA-Kristalle auch das in den Kristallen eingelagerte Lösungsmittel 1 freigesetzt und kann anschließend mit einem Gas aus der TEDA-Lösung herausgestrippt werden.

Nach der Fest-Flüssig-Trennung wird TEDA mit dem noch enthaltenen Lösungsmittel 1 in einem Mischapparat (z.B. Rührbehälter) intensiv mit dem jeweiligen Lösungsmittel 2 in Kontakt gebracht und gelöst.

Bevorzugt wird hierbei die Temperatur beim Auflösen des TEDAs auf kleiner/gleich 100°C, bevorzugt 20 bis 60°C, eingestellt. Der Absolutdruck beträgt hierbei bevorzugt 0,1 bis 5 bar, besonders 0,5 bis 1,5 bar.

Bei dem Lösungsmittel 2 handelt es sich bevorzugt um einen Alkohol, insbesondere C₂. ₁₃-Alkohol, und/oder Alkylether, insbesondere C₂₋₁₇-Alkylether.

Bei dem Lösungsmittel 2 handelt es sich bevorzugt um ein C₂₋₉-Diol, C₂₋₉-Diol-mono(C₁₋₄-alkyl)ether, C₂₋₉-Diol-di(C₁₋₄-alkyl)ether, C₃₋₈-Triol, C₂₋₈-Dialkylether und/oder C₃₋₁₂-alicyclischen Ether, insbesondere um ein C₂₋₆-Diol, C₂₋₆-Diol-mono(C₁₋₄-atkyl)ether, C₂₋₆-Diol-di(C₁₋₄-alkyl)ether. C₃₋₆-Triol, C₂₋₆-Dialkylether und/oder C₄₋₆-alicyclischen Ether, mit C₁₋₄-Alkyl = Methyl, Ethyl, n-Propyl, iso-Propyl, n-Butyl, iso-Butyl, tert.-Butyl.

Ganz besonders bevorzugte Lösungsmittel 2 sind Dipropylenglykol (DPG) (z.B. als Isomerengemisch), Monoethylenglykol (MEG), Diethylenglykol (DEG), Triethylenglykol (TEG), Tripropylenglykol (z.B. als Isomerengemisch), 1,4-Butandiol (BDO), 1,5-Pentandiol, 1,6-Hexandiol, 1,3-Propandiol, 1,2-Propandiol, 2,2-Dimethyl-1,3-propandiol (Neopentylglykol),
Dipropylenglykol-mono-n-butylether, Dipropylenglykol-mono-tert.-butylether, Dipropylenglykol-mono-methylether, Monoethylenglykol-mono-methylether, Monoethylenglykol-mono-ethylether, Monoethylenglykol-mono-n-butylether, Diethylenglykol-mono-methylether, Tripropylenglykol-mono-methylether,
Monoethylenglykol-dimethylether, Monoethylenglykol-diethylether, Diethylenglykoldimethylether, Diethylenglykol-diethylether, Tripropylenglykol-dimethylether, Glycerin, 1,3,5-Pentantriol, 1,1,1-Trimethylolpropan (TMP, 1,1,1-Tris-(hydroxymethyl)-propan),
Diethylether, Di-n-butylether, Methyl-tert.-butylether, Tetrahydrofuran (THF), 1,4-Dioxan.

Während oder nach dem Lösevorgang wird das aus der Kristallisation stammende Lösungsmittel 1 im gleichen wie beim Lösen verwendeten oder einem anderen geeigneten Apparat (z.B.: Rührbehälter oder Kolonne) mit Gas aus der resultierenden Lösung herausgestrippt. Dabei findet ein Stoffaustausch statt. Das Lösungsmittel 1 wird bevorzugt mit dem Strippgas ausgetragen.

Überraschenderweise wurde gefunden, dass die für die Qualitätsminderung des TEDAs verantwortlichen Neben- und Zersetzungsprodukte ebenfalls bevorzugt mit dem Gas herausgestrippt werden.

Nach erfolgtem Strippvorgang kann die TEDA-Lösung zur weiteren Verwendung, z.B. als Verkaufsware, abgefüllt werden.

Bevorzugt beträgt die Farbzahl einer erfindungsgemäß erhaltenen, z.B. 33 Gew.-%igen, TEDA-Lösung, z.B. in Dipropylenglykol, kleiner 50 APHA, insbesondere kleiner 30 APHA (DIN-ISO 6271). Dabei weist eine solche Lösung bevorzugt einen Wassergehalt von kleiner 0,35 Gew.% und eine TEDA-Reinheit (berechnet ohne Lösungsmittel) von größer 99,90 Gew.-%, insbesondere größer 99,95 Gew.-%, auf.

Die erfindungsgemäß erhaltenen TEDA-Lösungen weisen keinen Geruch auf, der von Nebenkomponenten, insbesondere von cyclischen gesättigten 5-Ring-N-Heterocyclen oder anderen cyclischen gesättigten 6-Ring-N-Heterocyclen und/oder aromatischen 5-oder 6-Ring-N-Heterocyclen, herrührt.

Die erfindungsgemäß erhaltenen TEDA-Lösungen weisen bevorzugt einen Gehalt an Lösungsmittel 1 von kleiner 50 Gew.-ppm, insbesondere kleiner 20 Gew.-ppm, (bezogen auf TEDA) auf.

Das bei der Strippung austretende Strippgas, das neben dem Lösungsmittel 1 aus der Kristallisationsstufe noch TEDA enthält, kann in einem meist als Absorption (z.B. mit Wasser) gestalteten weiteren Trennschritt in TEDA und mit Lösungsmittel 1 beladenem Gas getrennt werden.
Das so zurückgewonnene TEDA kann wieder in den Prozess zurückgeführt werden.

Die erfindungsgemäße Abtrennung des aus der Kristallisation stammenden Lösungsmittels 1 mit einem Strippgas, kann nach den dem Fachmann geläufigen Verfahren und Bedingungen erfolgen.

Der Apparatetyp zur Strippung wird nach Zweckmäßigkeitsgesichtspunkten ausgewählt. Bevorzugt werden Behälter, Rührbehälter oder Kolonnen mit oder ohne Einbauten eingesetzt. Die TEDA-Lösung kann kontinuierlich oder diskontinuierlich vorgelegt werden. Das Begasen der TEDA-Lösung mit Strippgas (Strippung) kann während oder nach dem Lösevorgang des TEDAs im Lösungsmittel 2 erfolgen.

Die Gaseinleitung erfolgt bevorzugt über eine Ringdüse, die sich bevorzugt am Boden des Behälters befindet.
Solche Ringdüsen sind dem Fachmann bekannt, z.B. aus Z. Gao et al., Chemical Engineering Research and Design: 4th International Symposium on Mixing in Industrial Processes (ISMIP-4), 14-16 May 2001, Toulouse, France / Joel Bertrand, Ed. (2001) 79(A8), 973-978, und Z.D. Chen et al., Chem. Eng. Res. Des. (1999) 77(A2), 104-109, und W.-M. Lu et al., Chem. Eng. J. (Lausanne) (1986) 33(2), 57-62.

Bevorzugt werden Gasgeschwindigkeiten im Bereich von 0,1 bis 5 cm/s, insbesondere 0,2 bis 1 cm/s, (bezogen auf den freien Behälterquerschnitt) eingestellt.

Bezüglich der Menge des verwendeten Strippgases wird bevorzugt so verfahren, dass, je nach Menge des Strippgases zu Beginn der Strippung, Abgas mit einer Konzentration des aus der Kristallisation stammenden Lösungsmittels 1 von ca. 0,1 bis 40 Gew.-%, bevorzugt 1 bis 10 Gew.-%, erhalten wird.

Als Strippgas eignen sich besonders Edelgase (Helium, Neon, Argon, Xenon), Edelgas-haltige Gase, Stickstoff, Stickstoff-haltige Gase, insbesondere Stickstoff-haltige Gase mit einem überwiegenden Anteil an N₂, wie z.B. Luft.

Ganz besonders bevorzugt wird Stickstoff, insbesondere N₂ in einer Reinheit größer 99 Vol.%, ganz besonders größer 99,9 Vol.%.

Die Strippung erfolgt bevorzugt über einen Zeitraum von 0,1 bis 5 Stunden, insbesondere von 1 bis 3 Stunden.

Die nach der Strippung resultierende TEDA-Lösung enthält im erfindungsgemäßen Verfahren weniger als 1 Gew.-%, bevorzugt weniger als 0,1 Gew.-%, ganz besonders weniger als 0,01 Gew.%, des aus der Kristallisation stammenden Lösungsmittels 1.

Bevorzugt wird die Temperatur bei der Strippung auf kleiner/gleich 100°C, besonders 20 bis 70°C, besonders bevorzugt 40 bis 60°C, eingestellt.
Der Absolutdruck beträgt hierbei bevorzugt 0,1 bis 5 bar, insbesondere 0,5 bis 1,5 bar.

Durch das erfindungsgemäße Verfahren werden infolge der Ausschleusung des aus der Kristallisation enthaltenen Lösungsmittels 1 und durch die Rückgewinnung des TEDAs in der Absorptionsstufe die TEDA-Verluste reduziert und reines TEDA mit der aufgabengemäß verbesserten Ausbeute und Reinheit erhalten.

Das im erfindungsgemäßen Verfahren eingesetzte und zu verdampfende TEDA kann nach den bekannten Verfahren, z.B. durch Umsetzung von Monoethanolamin, Diethanolamin, Triethanolamin, Ethylendiamin (EDA), Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator [z.B. Metallpyrophosphate, Metallphosphate (wie Erdalkalimonohydrogenphosphat), Zeolithe, Zirkoniumphosphate, Al₂O₃, SiO₂, phosphorhaltiges TiO₂ oder ZrO₂] bei erhöhter Temperatur (bevorzugt 250 bis 450°C), erhalten werden. Üblicherweise beträgt hierbei der Absolutdruck 0,1 bis 50 bar, insbesondere 0,1 bis 5 bar. Optional kann die Umsetzung in Gegenwart eines inerten polaren aprotischen Lösungsmittels (z.B. N-Alkylpyrrolidon (wie N-Methylpyrrolidon), Dioxan, THF, Dialkylformamid (wie Dimethylformamid), Dialkylacetamid (wie Dimethylacetamid)) und eines inerten Trägergases (z.B. N₂ oder Ar) durchgeführt werden.

Derartige Verfahren sind z.B. neben den eingangs zitierten Schriften beschrieben in DT-A-24 42 929, US-A-3,297,701, DE-A-36 34 258, DE-A-1 745 627, DE-A-37 18 395, EP-A-111 928, EP-A-842 935, EP-A-831 096, EP-A-952 152 und US-A-5,741,906.

Bevorzugt wird das zu verdampfende TEDA kontinuierlich gemäß WO-A-01/02404 aus EDA unter Einsatz eines Zeolith-Katalysators bei einer Reaktionstemperatur im Bereich von 310-370°C hergestellt, wobei der Eduktstrom 5-80, insbesondere 20-70, ganz besonders 35-60, Gew.-% EDA enthält, einen Wassergehalt von 2-60, insbesondere 10-60, Gew.-% aufweist und der Zeolith vom Pentasil-Typ ist, ein Si:AI-Atomverhältnis von 100-700:1 aufweist und zumindest teilweise in der H⁺ - und/oder NH₄⁺ - Form vorliegt.

Ganz besonders bevorzugt enthält der Eduktstrom zusätzlich 1-50, insbesondere 10-30, Gew.-% PIP.

Bevorzugt wird im erfindungsgemäßen Verfahren das TEDA aus einer Mischung enthaltend ein Lösungsmittel oder ein Verdünnungsmittel, wobei das Lösungs- oder Verdünnungsmittel bei Normaldruck (= 1,01325 bar) einen Siedepunkt im Bereich von 175 bis 250°C aufweist, verdampft und das dampfförmige TEDA in ein flüssiges Lösungsmittel 1 einleitet.

Bei dem Lösungsmittel, in dessen Gegenwart das TEDA verdampft wird und dem Lösungsmittel, in das das dampfförmige TEDA eingeleitet wird, kann es sich um das gleiche oder auch um verschiedene Lösungsmittel handeln.

Durch die Einleitung des dampfförmigen TEDAs in ein flüssiges Lösungsmittel (TEDA-Quench) wird die Bildung von unerwünschten Neben- und Zersetzungsprodukten, die zur Qualitätsminderung des TEDAs führen, verringert.
Der flüssige Aggregatzustand des TEDAs am Ausgang der Verdampfungsapparatur, z.B. Rektifikations- oder Destillationsapparatur, wird vermieden, die bei Destillationen übliche Verflüssigung des Destillats findet nicht statt. Das dampfförmige TEDA wird stattdessen direkt in ein flüssiges Lösungsmittel eingeleitet.

Das Lösungs- oder Verdünnungsmittel, das die Mischung enthält, aus der das TEDA verdampft wird, weist bevorzugt bei Normaldruck einen Siedepunkt im Bereich von 175 bis 250°C, besonders im Bereich von 180 bis 230°C, insbesondere im Bereich vom 190 bis 210°C, auf.

Als Lösungs- oder Verdünnungsmittel, das die Mischung enthält, aus der das TEDA verdampft wird, eignen sich besonders inerte
- polare aprotische Lösungsmittel [z.B. Alkyl-2-pyrrolidone (wie N-Methyl-2-pyrrolidon (NMP), 1-Ethyl-2-pyrrolidon (NEP), 1,5-Dimethyl-2-pyrrolidon, 1-Isopropyl-2-pyrrolidon), Ether (wie Diethylenglykoldiethylether, Triethylenglykoldimethylether, Triethylenglykoldiethylether), Ketone (wie Acetophenon, Propiophenon), Lactone (wie γ-Butyrolacton), Sulfoxide (wie Dimethylsulfoxid), Carbonsäureester (wie Fumarsäuredimethylester), Nitrile (wie Benzonitril) und Harnstoffe (wie 1,3-Dimethyl-imidazolidin-2-on (DMEU), Tetramethylharnstoff)],
- cyclische oder acyclische Kohlenwasserstoffe, insbesondere gesättigte cyclische oder acyclische Kohlenwasserstoffe (z.B. Undecan, Dodecan, cis-Dekalin, trans-Decalin),
- chlorierte aliphatische Kohlenwasserstoffe (z.B. 1-Chloroctan, 1,1-Dichloroctan),
- aromatische Kohlenwasserstoffe, Nitroaromaten und Phenole (z.B. Naphthalin, n-Butylbenzol, Phenol, Kresol, Nitrobenzol, Nitrophenol),
- chlorierte aromatische Kohlenwasserstoffe (z.B. 1,2-Dichlorbenzol, Benzylchlorid, 1,2,3,4-Tetramethylbenzol, 1,2,3,5-Tetramethylbenzol),
- Alkohole (z.B. Benzylalkohol, 2-Ethylhexanol, 1-Octanol, i-Decanol, 1,2-Propandiol, 1,3-Propandiol, Ethylenglykol, Diethylenglykol, 1,2-Propylenglykol, 1,3-Propylenglykol, 1,2-Butandiol, 1,3-Butandiol, 2,3-Butandiol, 1,4-Butandiol, Neopentylglykol, Diethylenglykolmonomethylether, Dipropylenglykol),
- primäre, sekundäre und tertiäre Amine (z.B. Tri-n-butylamin, Benzylamin, Anilin, N-Ethylanilin, N,N-Dimethylanilin, N,N-Diethylanilin),
- N-Alkylamide (z.B. N-Methylformamid, N-Methylacetamid)
und deren Gemische.

Besonders bevorzugt sind polare aprotische Lösungs- oder Verdünnungsmittel mit einem E^{N}_{T}-Wert von 0,1 bis 0,6, besonders von 0,2 bis 0,5, insbesondere von 0,3 bis 0,45.
(Zur Definition des E^{N}_{T}-Wertes siehe Ch. Reichardt, Solvents and solvent effects in organic chemistry, 2. Auflage, VCH 1988).

Ganz besonders bevorzugte Lösungsmittel sind hier NMP und Monoethylenglykol.

Das Lösungs- oder Verdünnungsmittel, das die Mischung enthält, aus der das TEDA verdampft wird, wird bevorzugt nach der Synthese des TEDAs dem rohen oder dem noch verunreinigten TEDA zugesetzt. Vorteilhaft ist die Zugabe des Lösungsmittels in den Sumpf der Kolonne der TEDA-Destillation.

Das Lösungs- oder Verdünnungsmittel kann im einmaligen Durchlauf oder nach der Abtrennung der Schwersieder als Kreislauflösung eingesetzt werden.

Bevorzugt wird so verfahren, dass, je nach Art des Lösungs- oder Verdünnungsmittels, Lösungen oder Mischungen mit einem TEDA-Gehalt von ca. 1 bis 90 Gew.%, bevorzugt 40 bis 70 Gew.%, erhalten werden.

Die Verdampfung des TEDAs, gegebenenfalls aus der Mischung enthaltend ein Lösungs- oder Verdünnungsmittel, kann nach den dem Fachmann geläufigen Verfahren und Bedingungen erfolgen, z.B. durch einfache Verdampfung aus rohem TEDA (Sumpfverdampfung) oder bevorzugt in einer Destillations- oder Rektifikationsapparatur, wobei das TEDA oder ein Gemisch enthaltend das TEDA (rohes TEDA), optional zusammen mit dem Lösungs- oder Verdünnungsmittel, vorgelegt wird.

Bevorzugt wird das dampfförmige TEDA am Kopf oder in einem Seitenabzug einer Destillationskolonne erhalten. Das dampfförmige TEDA besitzt bevorzugt eine Reinheit von größer 90 Gew.-%. besonders größer 95 Gew.-%, insbesondere größer 97 Gew.-%.

Vorteilhaft wird bei der destillativen Aufarbeitung des TEDAs durch konstruktive Maßnahmen an Kolonnen und/oder Verdampfern (z.B.: Minimierung des Sumpfraumes) und/oder durch den Einsatz von thermisch schonenden Eindampfverfahren (z.B.: Fallfilmverdampfer, Dünnschichtverdampfer) die Verweilzeit und dadurch die thermische Belastung gering gehalten.

Bevorzugt wird die Temperatur der Mischung, enthaltend TEDA und das Lösungs- oder Verdünnungsmittel, aus der das TEDA verdampft wird (z.B. des Sumpfes der entsprechenden TEDA-Destillationskolonne), durch Wahl des einzusetzenden Lösungs- oder Verdünnungsmittels, des TEDA-Gehalts der Mischung und/oder des Drucks, auf ≤ 230°C, bevorzugt 190 bis 210°C eingestellt. Der Absolutdruck beträgt hierbei bevorzugt 0,1 bis 5 bar, besonders 0,5 bis 1,5 bar.

Die Zeitdauer zwischen Anfall des dampfförmigen TEDAs und Quench des TEDAs beträgt vorteilhaft ≤ 10 Sekunden.

Als Lösungsmittel 1 für den TEDA-Quench eignen sich besonders cyclische oder acyclische (= aliphatische) Kohlenwasserstoffe (insbesondere verzweigte oder unverzweigte Alkane oder Alkangemische), wie z.B. n-Pentan, iso-Pentan, Cyclopentan, n-Hexan, Cyclohexan, n-Heptan, n-Octan, Petrolether (PE) (z.B. PE 30/60, 35/60, 40/60), chlorierte aliphatische Kohlenwasserstoffe (insbesondere chlorierte Alkane, wie z.B. Dichlormethan, Trichlormethan, Dichlorethan, Trichlorethan),
aromatische Kohlenwasserstoffe (wie z.B. Benzol, Toluol, Xylole), chlorierte aromatische Kohlenwasserstoffe (wie z.B. Chlorbenzol),
Alkohole, insbesondere C₁₋₄-Alkohole (wie z.B. Methanol, Ethanol, n-Propanol), Ketone, insbesondere C₃₋₆-Ketone, (insbesondere aliphatische. Ketone, wie Aceton, Methylethylketon, Diethylketon),
aliphatische Carbonsäureester (wie z.B. Essigsäuremethylester, Essigsäureethylester), aliphatische Nitrile (wie z.B. Acetonitril, Propionitril),
Ether (wie z.B. Dioxan, THF, Diethylether, Methyl-tert.-butylether, Ethylenglykoldimethylether)
und deren Gemische.

Als Lösungsmittel 1 für den TEDA-Quench wird bevorzugt ein aliphatischer Kohlenwasserstoff, insbesondere ein gesättigter aliphatischer Kohlenwasserstoff mit 5 bis 8 C-Atomen (wie z.B. n-Pentan, iso-Pentan, Cyclopentan, n-Hexan, Cyclohexan, n-Heptan oder Gemische hiervon, wie z.B. Petrolether), Ethanol, Toluol und/oder Aceton verwendet.

Die Kristallisation des reinen TEDAs aus der erhaltenen TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von bevorzugt mindestens 99,5 Gew.-%, insbesondere mindestens 99,9 Gew.-%) und die Farbzahl einer 33 Gew.-%igen Lösung, z.B. in Dipropylenglykol, beträgt bevorzugt kleiner 50, insbesondere kleiner 30, APHA (DIN-ISO 6271).

Die Einleitung des dampfförmigen TEDAs in das flüssige Lösungsmittel erfolgt in einem Quenchapparat, z.B. bevorzugt in einem Fallfilmkondensator (Dünnschicht-, Rieselfilm-oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Das Lösungsmittel für den TEDA-Quench kann im einmaligen Durchlauf oder als Kreislauflösung eingesetzt werden.

Bezüglich der Menge des verwendeten Lösungsmittels 1 im TEDA-Quench wird bevorzugt so verfahren, dass, je nach Art des Lösungsmittels, Lösungen mit einem TEDA-Gehalt von ca. 1 bis 50 Gew.-%, bevorzugt 20 bis 40 Gew.-%, erhalten werden.

Bevorzugt wird die Temperatur im TEDA-Quench durch Temperierung des eingesetzten Lösungsmittels und/oder des Quenchapparates auf 20 bis 100°C, bevorzugt 30 bis 60°C, eingestellt.

Der Absolutdruck im TEDA-Quench beträgt bevorzugt 0,5 bis 1,5 bar.

Durch die partielle Verdampfung des eingesetzten Lösungsmittels im TEDA-Quench infolge der Wärmezufuhr des dampfförmigen TEDAs ist der Gasraum im Quenchapparat mit Lösungsmitteldampf gesättigt. Dadurch wird/werden die Desublimation des dampfförmigen TEDAs und die dadurch bedingten Verstopfungsprobleme durch Feststoffablagerung im Apparat und/oder in den Austragsleitungen deutlich reduziert oder völlig verhindert.

Gemäß einer bevorzugten Ausführungsform lässt sich das erfindungsgemäße Verfahren wie folgt ausführen:

Eine Mischung enthaltend TEDA, die z.B. als Reaktionsaustrag in einem kontinuierlichen Verfahren durch Umsetzung von Ethylendiamin und Piperazin in einem Gasphasenreaktor bei 320 bis 420°C und 0,5 bis 1,5 bar (abs.) in Gegenwart eines Lösungsmittels (z.B. Wasser), eines Trägergases (z.B. N₂ oder Ar) und eines Zeolithkatalysators erhalten wurde (z.B. gemäß WO-A-01/02404), wird in eine Destillationsapparatur mit einer Destillationskolonne mit z.B. ca. 15 theoretischen. Stufen geleitet. Hier werden Leichtsieder (wie z.B. Ammoniak, Ethylamin, Wasser) bei einer Kopftemperatur von 95 bis 120°C und einem Absolutdruck von bevorzugt 500 mbar bis 1,5 bar über Kopf abgetrennt. Der Sumpfablauf wird in eine weitere Destillationskolonne mit z.B. ca. 30 theoretischen Stufen gepumpt. Bei einer Kopftemperatur von 140 bis 160°C und einem Absolutdruck von bevorzugt 500 mbar bis 1,5 bar wird in dieser Kolonne Piperazin über Kopf abgetrennt und optional wieder zurück zum Synthesereaktor gefahren.

Der Sumpfablauf enthaltend TEDA und Schwersieder wird in eine weitere Destillationskolonne mit z.B. ca. 25 theoretischen Stufen gepumpt. Bei einem Absolutdruck von bevorzugt 500 mbar bis 1,5 bar werden in dieser Kolonne die Schwersieder über den Sumpfablauf ausgeschleust. Am Kopf der Kolonne wird TEDA mit einer Reinheit von größer 95 Gew.-%, insbesondere größer 97 Gew.-%, über einen Teilkondensator dampfförmig abgezogen und in einem Fallfilmkondensator in einem Lösungsmittel 1 (z.B. Pentan, Cyclohexan) bei einer Temperatur von bevorzugt 30 bis 100°C, besonders 30 bis 60°C, direkt schockartig abgekühlt und gleichzeitig gelöst (TEDA-Quench).

Nach dem TEDA-Quench wird TEDA aus der Lösung in einer Kristallisationsstufe durch Verdampfen des Lösungsmittels bei einer Temperatur von bevorzugt 10 bis 100°C, besonders 20 bis 40°C, und bei einem Druck von bevorzugt 0,1 bis 5 bar, besonders 0,5 bis 1,5 bar, oder durch Abkühlen bei einer Temperatur von bevorzugt -10 bis 40°C, besonders 0 bis 10°C, auskristallisiert.

Die aus dem Kristaller abgezogene Suspension wird in einer Fest-Flüssig-Trennung, z.B. in einer Zentrifuge, in TEDA und Mutterlauge getrennt. Die Mutterlauge, die noch Reste des Wertprodukts enthält, wird nun in einer Extraktionsstufe (z.B. Extraktionskolonne) bei einer Temperatur von bevorzugt 10 bis 100°C, besonders 20 bis 40°C, und bei einem Absolutdruck von bevorzugt 0,1 bis 5 bar, besonders 0,5 bis 1,5 bar, intensiv mit einem Extraktionsmittel (z.B. Wasser) in Kontakt gebracht.

Die nach dem Stoffaustausch und der Phasentrennung aus der Extraktionsstufe austretende Extraktphase, die den Hauptanteil des TEDAs und die unerwünschten Neben-und Zersetzungsprodukte, die zu einer Qualitätsminderung des TEDAs führen, enthält, wird zurück zum. Reaktor oder in die Destillation geleitet. Die Raffinatphase, die nur noch Spuren des TEDA enthält, wird zurück in den TEDA-Quench geleitet.

Das aus der Fest-Flüssig-Trennung erhaltene TEDA, das noch Reste des bei der Kristallisation verwendeten Lösungsmittels 1 (z.B. Pentan, Cyclohexan) sowie die für die Qualitätsminderung des TEDAs verantwortlichen Neben- und Zersetzungsprodukte enthält, wird in einem Mischapparat (z.B. Rührbehälter) in einem Lösungsmittel 2 (z.B. Dipropylenglykol, Monoethylenglykol oder 1,4-Butandiol) gelöst. Bevorzugt wird die Temperatur beim Lösen des TEDAs auf kleiner/gleich 100°C, besonders 20 bis 60°C eingestellt. Der Absolutdruck beträgt hierbei bevorzugt 0,1 bis 5 bar, besonders 0,5 bis 1,5 bar.

Zur Abtrennung des Lösungsmittels 1 mit einem Strippgas wird bei einer Temperatur im Bereich von bevorzugt 10 bis 100°C, besonders 20 bis 70°C, insbesondere 40 bis 60°C, und bei einem Absolutdruck von bevorzugt 0,1 bis 5 bar, besonders 0,5 bis 1,5 bar, intensiv mit einem Gas (z.B. Stickstoff) in Kontakt gebracht (Strippung). Dabei wird das Lösungsmittel 1 sowie die unerwünschten Nebenkomponenten bevorzugt vom Strippgas aufgenommen und von der TEDA-Lösung abgetrennt.

### Beispiele

### Beispiel 1 (Vergleichsbeispiel):

Das aus der Fest-Flüssig-Trennung erhaltene TEDA mit einem Pentangehalt (Lösungsmittel 1) von ca. 2000 Gew.-ppm wurde in einem Rotavapor bei 45°C / 1 bar_{abs.} etwa zwei Stunden getrocknet. Dabei konnte der Pentangehalt nur auf ca. 400 Gew.-ppm reduziert werden. Der TEDA-Verlust betrug dabei ca. 10 Gew.-%. Eine Verlängerung der Trockenzeit und/oder eine Erhöhung der Temperatur führte zu keiner merklichen Reduzierung des Pentangehaltes aber zu deutlich höheren TEDA-Verlusten.

### Beispiel 2 (erfindungsgemäß):

Die Durchführung des Versuchs erfolgte wie in Beispiel 1 beschrieben, jedoch wurde nach der Fest-Flüssig-Trennung das erhaltene TEDA in einem Rührbehälter in Dipropylenglykol (= Lösungsmittel 2) bei 50°C gelöst und anschließend mit Stickstoff (700 Liter/h) bei 50°C gestrippt. Die Gaseinleitung erfolgte über eine Ringdüse am Boden des Behälters mit einer Gasgeschwindigkeit von 0,6 cm/s (bezogen auf den freien Behälterquerschnitt). Nach einer Strippdauer von zwei Stunden lag der Pentangehalt (= Lösungsmittel 1) unter 10 Gew.-ppm (bezogen auf TEDA). Der TEDA-Verlust betrug < 1 Gew.-%.

## Patentansprüche

1. Verfahren zur Herstellung einer Lösung von reinem Triethylendiamin (TEDA), in dem man TEDA verdampft, das dampfförmige TEDA in ein flüssiges Lösungsmittel 1 einleitet (Quench) und das TEDA aus der erhaltenen Lösung auskristallisiert und abtrennt (Fest-Flüssig-Trennung), **dadurch gekennzeichnet, dass** man das erhaltene kristalline TEDA in einem Lösungsmittel 2 löst und durch die resultierende Lösung ein Strippgas leitet (Strippung), wobei Lösungsmittel 1 freigesetzt und anschließend mit einem Gas aus der TEDA-Lösung herausgestrippt wird.

2. Verfahren nach Anspruch 1, **dadurch gekennzeichnet, dass** es sich bei dem Strippgas um Stickstoff, ein Edeigas, ein Stickstoff-haltiges Gas oder ein Edelgas-haitiges Gas handelt.

3. Verfahren nach den Ansprüchen 1 oder 2, **dadurch gekennzeichnet, dass** es sich bei dem Lösungsmittel 2 um einen Alkohol und/oder Alkylether handelt.

4. Verfahren nach den Ansprüchen 1 oder 2, **dadurch gekennzeichnet, dass** es sich bei dem Lösungsmittel 2 um ein C₂₋₉-Diol, C₂₋₉-Diol-mono(C₁₋₄-alkyl)ether, C₂₋₉-Diol-di(C₁₋₄-alkyl)ether, C₃₋₈-Triol, C₂₋₈-Dialkylether und/oder C₃₋₁₂-alicyclischen Ether handelt.

5. Verfahren nach den Ansprüchen 1 oder 2, **dadurch gekennzeichnet, dass** es sich bei dem Lösungsmittel 2 um Dipropylenglykol, Monoethylenglykol und/oder 1,4-Butandiol handelt.

6. Verfahren nach einem der vorhergehenden Ansprüche, **dadurch gekennzeichnet, dass** die Strippung bei einer Temperatur im Bereich von 20 bis 100°C erfolgt.

7. Verfahren nach einem der Ansprüche 1 bis 5, **dadurch gekennzeichnet, dass** die Strippung bei einer Temperatur im Bereich von 40 bis 60°C erfolgt.

8. Verfahren nach einem der vorhergehenden Ansprüche, **dadurch gekennzeichnet, dass** die Strippung bei einem Absolutdruck im Bereich von 0,1 bis 5 bar erfolgt.

9. Verfahren nach einem der vorhergehenden Ansprüche, **dadurch gekennzeichnet, dass** die Einleitung des Strippgases über eine Ringdüse erfolgt.

10. Verfahren nach einem der vorhergehenden Ansprüche, **dadurch gekennzeichnet, dass** die Strippgasgeschwindigkeit 0.1 bis 5 cm/s (bezogen auf den freien Behälterquerschnitt) beträgt.

11. Verfahren nach einem der vorhergehenden Ansprüche, **dadurch gekennzeichnet, dass** das bei der Strippung anfallende Abgas bezüglich des Lösungsmittels 1 eine Konzentration im Bereich von 0,1 bis 40 Gew.-% aufweist.

12. Verfahren nach einem der vorhergehenden Ansprüche, **dadurch gekennzeichnet, dass** im bei der Strippung anfallenden Abgas enthaltenes TEDA durch Absorption zurückgewonnen wird.

13. Verfahren nach einem der vorhergehenden Ansprüche, **dadurch gekennzeichnet, dass** die nach der Strippung resultierende TEDA-Lösung einen Gehalt an Lösungsmittel 1 von kleiner 1 Gew.-% aufweist.

14. Verfahren nach einem der Ansprüche 1 bis 12, **dadurch gekennzeichnet, dass** die nach der Strippung resultierende TEDA-Lösung einen Gehalt an Lösungsmittel 1 von kleiner 0,1 Gew.-% aufweist.

15. Verfahren nach einem der vorhergehenden Ansprüche, **dadurch gekennzeichnet, dass** man das dampfförmige TEDA am Kopf oder in einem Seitenabzug einer Destillationskolonne erhält.

16. Verfahren nach einem der vorhergehenden Ansprüche, in dem man das TEDA aus einer Mischung enthaltend ein Lösungsmittel oder ein Verdünnungsmittel, wobei das Lösungs- oder Verdünnungsmittel bei Normaldruck einen Siedepunkt im Bereich von 175 bis 250°C aufweist, verdampft.

17. Verfahren nach einem der vorhergehenden Ansprüche, **dadurch gekennzeichnet, dass** das flüssige Lösungsmittel 1 aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt wird.

18. Verfahren nach einem der Ansprüche 1 bis 16, **dadurch gekennzeichnet, dass** man als flüssiges Lösungsmittel 1 (Quenchlösungsmittel) n-Pentan, iso-Pentan, n-Hexan, Cyclohexan, Petrolether, Ethanol, Aceton, Toluol oder Gemische hiervon einsetzt.

19. Verfahren nach einem der vorhergehenden Ansprüche, **dadurch gekennzeichnet, dass** das dampfförmige TEDA zur Einleitung in das flüssige Lösungsmittel 1 (Quench) eine Reinheit von größer 95 Gew.-% aufweist.

20. Verfahren nach einem der vorhergehenden Ansprüche, **dadurch gekennzeichnet, dass** das zu verdampfende TEDA durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator bei erhöhter Temperatur erhalten wurde.

21. Verfahren nach dem vorhergehenden Anspruch, **dadurch gekennzeichnet, dass** es sich bei dem Katalysator um ein Metallphosphat oder einen Zeolith handelte.

22. Verfahren nach einem der beiden vorhergehenden Ansprüche, **dadurch gekennzeichnet, dass** man die Umsetzung bei einer Temperatur im Bereich von 250 bis 450°C in der Gasphase durchführte.

23. Verfahren nach einem der Ansprüche 1 bis 19, **dadurch gekennzeichnet, dass** das zu verdampfende TEDA kontinuierlich aus EDA unter Einsatz eines Zeolith-Katalysators bei einer Reaktionstemperatur im Bereich von 310-370°C erhalten wurde, wobei der Eduktstrom 20-70 Gew.-% EDA enthält, einen Wassergehalt von 10-60 Gew.-% aufweist und der Zeolith vom Pentasil-Typ ist, ein Si:Al-Atomverhältnis von 100-700:1 aufweist und zumindest teilweise in der H⁺ und/oder NH₄⁺ - Form vorliegt.

24. Verfahren nach einem der vorhergehenden Ansprüche zur Herstellung einer Lösung von TEDA mit einer TEDA-Reinheit (berechnet ohne Lösungsmittel) von größer 99,90 Gew.-%.

25. Verfahren nach einem der vorhergehenden Ansprüche zur Herstellung einer Lösung von TEDA mit einem Gehalt an Lösungsmittel 1 von kleiner 50 Gew.-ppm (bezogen auf TEDA).

## Claims

1. A process for preparing a solution of pure triethylenediamine (TEDA), in which TEDA is vaporized, the gaseous TEDA is passed into a liquid solvent 1 (quench) and the TEDA is crystallized from the resulting solution and separated off (solid-liquid separation), wherein the crystalline TEDA obtained is dissolved in a solvent 2 and a stripping gas is passed through the resulting solution (stripping), with solvent 1 being liberated and subsequently stripped from the TEDA solution by means of a gas.

2. The process according to claim 1, wherein the stripping gas is nitrogen, a noble gas, a nitrogen-containing gas or a gas comprising noble gas.

3. The process according to claim 1 or 2, wherein the solvent 2 is an alcohol and/or alkyl ether.

4. The process according to claim 1 or 2, wherein the solvent 2 is a C₂₋₉-diol, C₂₋₉-diol mono(C₁₋₄-alkyl) ether, C₂₋₉-diol di (C₁₋₄-alkyl) ether, C₃₋₈-triol, C₂₋₈-dialkyl ether and/or C₃₋₁₂-alicyclic ether.

5. The process according to claim 1 or 2, wherein the solvent 2 is dipropylene glycol, monoethylene glycol and/or 1,4-butanediol.

6. The process according to any of the preceding claims, wherein stripping is carried out at a temperature in the range from 20 to 100°C.

7. The process according to any of claims 1 to 5, wherein stripping is carried out at a temperature in the range from 40 to 60°C.

8. The process according to any of the preceding claims, wherein stripping is carried out at an absolute pressure in the range from 0.1 to 5 bar.

9. The process according to any of the preceding claims, wherein the stripping gas is introduced via a ring nozzle.

10. The process according to any of the preceding claims, wherein the stripping gas velocity is from 0.1 to 5 cm/s (based on the free cross section of the vessel).

11. The process according to any of the preceding claims, wherein the offgas obtained in stripping has a concentration of the solvent 1 in the range from 0.1 to 40% by weight.

12. The process according to any of the preceding claims, wherein TEDA present in the offgas obtained in stripping is recovered by absorption.

13. The process according to any of the preceding claims, wherein the TEDA solution resulting after stripping has a content of solvent 1 of less than 1% by weight.

14. The process according to any of claims 1 to 12, wherein the TEDA solution resulting after stripping has a content of solvent 1 of less than 0.1% by weight.

15. The process according to any of the preceding claims, wherein the gaseous TEDA is obtained at the top or at a side offtake of a distillation column.

16. The process according to any of the preceding claims in which the TEDA is vaporized from a mixture comprising a solvent or diluent having a boiling point at atmospheric pressure in the range from 175 to 250°C.

17. The process according to any of the preceding claims, wherein the liquid solvent 1 is selected from the group consisting of cyclic and acyclic hydrocarbons, chlorinated aliphatic hydrocarbons, aromatic hydrocarbons, alcohols, ketones, aliphatic carboxylic esters, aliphatic nitriles and ethers.

18. The process according to any of claims 1 to 16, wherein the liquid solvent 1 (quench solvent) used is n-pentane, isopentane, n-hexane, cyclohexane, petroleum ether, ethanol, acetone, toluene or a mixture thereof.

19. The process according to any of the preceding claims, wherein the gaseous TEDA to be passed into the liquid solvent 1 (quench) has a purity of greater than 95% by weight.

20. The process according to any of the preceding claims, wherein the TEDA to be vaporized has been obtained by reaction of monoethanolamine, diethanolamine, triethanolamine, ethylenediamine, diethylenetriamine, triethylenetetramine, piperazine, N-(2-hydroxyethyl)piperazine, N,N'-bis(2-hydroxyethyl)piperazine, N-(2-aminoethyl)piperazine, N,N'-bis(2-aminoethyl)piperazine, morpholine or mixtures thereof over a catalyst at elevated temperature.

21. The process according to the preceding claim, wherein the catalyst was a metal phosphate or a zeolite.

22. The process according to either of the two preceding claims, wherein the reaction was carried out in the gas phase at a temperature in the range from 250 to 450°C.

23. The process according to any of claims 1 to 19, wherein the TEDA to be vaporized has been obtained continuously from EDA using a zeolite catalyst at a reaction temperature in the range 310-370°C, with the feed stream comprising 20-70% by weight of EDA, having a water content of 10-60% by weight and the zeolite being of the pentasil type, having an Si:Al atomic ratio of 100-700:1 and being at least partly present in the H⁺ and/or NH₄⁺ form.

24. The process according to any of the preceding claims for preparing a solution of TEDA having a TEDA purity (calculated without solvent) of greater than 99.90% by weight.

25. The process according to any of the preceding claims for preparing a solution of TEDA having a content of solvent 1 of less than 50 ppm by weight (based on TEDA).

## Revendications

1. Procédé de production d'une solution de triéthylène diamine pure (TEDA), dans lequel on évapore la TEDA, on envoie la TEDA sous forme vapeur dans un solvant liquide 1 (quench) et on cristallise et on sépare la TEDA à partir de la solution obtenue (séparation solide-liquide), **caractérisé en ce que** l'on dissout la TEDA cristalline obtenue dans un solvant 2 et que l'on fait passer un gaz de stripage à travers la solution résultante (stripage), le solvant 1 étant libéré et ensuite strippé avec un gaz lors de la solution de TEDA.

2. Procédé selon la revendication 1, **caractérisé en ce que** le gaz de stripage est de l'azote, un gaz rare, un gaz contenant de l'azote ou un gaz contenant un gaz rare.

3. Procédé selon l'une quelconque des revendications 1 ou 2, **caractérisé en ce que** le solvant 2 est un alcool et/ou un éther d'alkyle.

4. Procédé selon l'une quelconque des revendications 1 ou 2, **caractérisé en ce que** le solvant 2 est un diol en C₂ à C₉, un (diol en C₂ à C₉)monoéther d' (alkyle en C₁ à C₄), un (diol en C₂ à C₉)diéther d' (alkyle en C₁ à C₄), un triol en C₃ à C₈, d'un éther de dialkyle en C₂ à C₈ et/ou un éther alicyclique en C₃ à C₁₂.

5. Procédé selon l'une quelconque des revendications 1 ou 2, **caractérisé en ce que** le solvant 2 est du dipropylène glycol, du monoéthylène glycol et/ou du 1,4-butane diol.

6. Procédé selon l'une quelconque des revendications précédentes, **caractérisé en ce que** le stripage s'effectue à une température dans l'intervalle de 20 à 100 °C.

7. Procédé selon l'une quelconque des revendications 1 à 5, **caractérisé en ce que** le stripage s'effectue à une température dans l'intervalle de 40 à 60 °C.

8. Procédé selon l'une quelconque des revendications précédentes, **caractérisé en ce que** le stripage s'effectue à une pression absolue dans l'intervalle de 0,1 à 5 bars.

9. Procédé selon l'une quelconque des revendications précédentes, **caractérisé en ce que** l'introduction du gaz de stripage s'effectue par une buse annulaire.

10. Procédé selon l'une quelconque des revendications précédentes, **caractérisé en ce que** la vitesse du gaz de stripage est de 0,1 à 5 cm/s (rapportée à la section libre du récipient).

11. Procédé selon l'une quelconque des revendications précédentes, **caractérisé en ce que** le gaz apparaissant lors du stripage présente une concentration en solvant 1 dans l'intervalle de 0,1 à 40 % en poids.

12. Procédé selon l'une quelconque des revendications précédentes, **caractérisé en ce que** la TEDA contenue dans le gaz apparaissant lors du stripage est récupérée par absorption.

13. Procédé selon l'une quelconque des revendications précédentes, **caractérisé en ce que** la solution de TEDA obtenue après le stripage présente une teneur en solvant 1 inférieure à 1 % en poids.

14. Procédé selon l'une quelconque des revendications 1 à 12, **caractérisé en ce que** la solution de TEDA obtenue après le stripage présente une teneur en solvant 1 inférieure à 0,1 % en poids.

15. Procédé selon l'une quelconque des revendications précédentes, **caractérisé en ce que** l'on obtient la TEDA sous forme vapeur en tête ou dans un prélèvement latéral d'une colonne de distillation.

16. Procédé selon l'une quelconque des revendications précédentes, dans lequel on évapore la TEDA à partir d'un mélange contenant un solvant ou un diluant, le solvant ou le diluant présentant à la pression normale un point d'ébullition dans l'intervalle de 175 à 250 °C.

17. Procédé selon l'une quelconque des revendications précédentes, **caractérisé en ce que** le solvant liquide 1 est choisi parmi le groupe des hydrocarbures cycliques ou acycliques, des hydrocarbures aliphatiques chlorés, des hydrocarbures aromatiques, des alcools, des cétones, des esters d'acides carboxyliques aliphatiques, des nitriles et des éthers aliphatiques.

18. Procédé selon l'une quelconque des revendications 1 à 16, **caractérisé en ce que** l'on utilise comme solvant liquide 1 (solvant de quench) du n-pentane, de l'isopentane, du n-hexane, du cyclohexane, de l'éther de pétrole, de l'éthanol, de l'acétone, du toluène ou des mélanges de ceux-ci.

19. Procédé selon l'une quelconque des revendications précédentes, **caractérisé en ce que** la TEDA sous forme vapeur à introduire dans le solvant liquide 1 (quench) présente une pureté de plus de 95 % en poids.

20. Procédé selon l'une quelconque des revendications précédentes, **caractérisé en ce que** la TEDA à évaporer a été obtenue par réaction à température accrue de monoéthanolamine, de diéthanolamine, de triéthanolamine, d'éthylène diamine, de diéthylène triamine, de triéthylène tétramine, de pipérazine, de N-(2-hydroxyéthyl)-pipérazine, de N,N'-bis(2-hydroxyéthyl)-pipérazine, de N-(2-aminoéthyl)-pipérazine, de N,N'-bis(2-aminoéthyl)-pipérazine, de morpholine ou de mélanges de celles-ci sur un catalyseur.

21. Procédé selon la revendication précédente, **caractérisé en ce que** le catalyseur est un phosphate métallique ou une zéolithe.

22. Procédé selon l'une quelconque des deux revendications précédentes, **caractérisé en ce que** l'on exécute la réaction à une température dans l'intervalle de 250 à 450 °C dans la phase gazeuse.

23. Procédé selon l'une quelconque des revendications 1 à 19, **caractérisé en ce que** la TEDA à évaporer a été obtenue continûment à partir de EDA en utilisant un catalyseur à zéolithe à une température de réaction dans l'intervalle de 310 à 370 °C, sachant que le flux de composé de départ contient 20 à 70 % en poids de EDA, qu'il présente une teneur en eau de 10 à 60 % en poids et que la zéolithe est de type pentasil, qu'elle présente un rapport atomique Si:Al de 100-700:1 et qu'elle se présente au moins en partie sous la forme H⁺ et/ou NH₄⁺.

24. Procédé selon l'une quelconque des revendications précédentes pour la production d'une solution de TEDA avec une pureté de la TEDA (calculée sans solvant) de plus de 99,90 % en poids.

25. Procédé selon l'une quelconque des revendications précédentes pour la production d'une solution de TEDA avec une teneur en solvant 1 de moins de 50 ppm en poids (rapportée à la TEDA).
